Europäisches Patentamt

European Patent Office

Office européen des brevets

(19)

(11) Publication number: **0 000 465**
**A1**

# EUROPEAN PATENT APPLICATION

(21) Application number: 78850007.2

(22) Date of filing: 06.07.78

(51) Int. Cl.²: **A61H5/00**

(30) Priority: 07.07.77 SE 7707915

(43) Date of publication of application: 24.01.79
Bulletin 79/2

(84) Designated Contracting States: **CH DE FR GB NL**

(71) Applicant: Brischewski, Alfred, Östra Gatan 21, 442 00
Kungälv (SE)
(84) Designated Contracting States: **CH DE FR GB NL**

(71) Applicant: Krus, Lennart Hilding, Trankärrsliden 16,
442 00 Kungälv (SE)
(84) Designated Contracting States: **CH DE FR GB NL**

(71) Applicant: Holmgren, Bernt, P.O. Box 2522, 430 34
Onsala (SE)
(84) Designated Contracting States: **CH DE FR GB NL**

(72) Inventor: Brischewski, Alfred, Östragatan 21, 442 00
Kungälv (SE)
Krus, Lennart Hilding, Trankärrsliden 16, 442 00
Kungälv (SE)
Holmgren, Bernt, P.O. Box 2522, 430 34 Onsala (SE)

(74) Representative: Wennborg, Göte, KRANSELL &
WENNBORG AB Sandhamnsgatan 42, 115 28
Stockholm (SE)

(54) An instrument for filling an injection syringe.

(57) An instrument for filling an injection syringe of non-recurrent or multiple-use type to be used in particular by a person with defective vision comprises a body portion (1) permitting securing of a medicament bottle (5) and an injection syringe (6), so that the needle of the syringe precisely hits and penetrates the cap of the bottle. An axially displaceable dosage rod (2) coacts with the syringe's plunger part (6b) so that filling of predetermined dose in the syringe is effected. A finger-actuated control knob (3) journalled at the body portion accomplishes via a worm gear (3b, 2b) the axial displacement of the dosage rod. The end (2a) of the dosage rod may be laterally resilient and possibly provided with a driving dog coacting with the end plate (6c) of the plunger part.

1

## An instrument for filling an injection syringe

The present invention concerns an instrument for filling an injection syringe of non-recurrent or multiple use type intended for use especially by a person with defective vision.

In US patent 3,610,241 there is described a previously known instrument of this kind designated "guide" and comprising a body portion having means for securing a medicament or drug bottle and a syringe so that the needle of the syringe precisely hits and penetrates the cap of the bottle, and having an axially movable dosage rod which by coaction with the plunger part of the syringe permits drawing in a predetermined volume into the syringe.

In this known device the dosage rod proper is directly finger-actuated which in practise will involve great risks of false dosage, especially when the syringe is manipulated by a person with defect vision. Further, the known instrument does not permit sufficient exactness in the dosing operation.

An object of the present invention is to provide an instrument of the abovementioned kind which by avoiding the drawbacks in instruments previously knwon permits accurate setting of an

2

ordinated dose and reliable filling of the syringe also by a person with defective vision.

The instrument according to the invention is substantially characterized by a finger-actuated control member which is rotatably journalled at the body portion and via a transmission means effects the displacement of the dosage rod.

Due to the fact that the dosage rod in the way mentioned is actuated by a special control member via a transmission means the risk is avoided that the rod shall take an undesired wrong position, for instance by hitting an item when being manu-pulated. The transmission means may namely contribute to automatically retain the dosage rod in the position set, so that it cannot be shifted therefrom. In addition the control member and the transmission means permit substantially greater exactness when setting the determined dose.

In practise it is preferred that the control member consists of a knob rotatable around an axis perpendicular to the axis of the syringe.

Such a control knob permits simple and reliable actuation of the dosage rod and may for this purpose have one or several clearly noticeable and/or audible markings per revolution which can be accomplished by a ball-latch or the like.

Further, it is preferred that the control knob via a worm gear actuates the dosage rod. At the same time such a worm gear form a reliable retaining means which secures the dosage rod in the position set.

In one embodiment the dosage rod is laterally resilient so that at filling of the syringe it permits transit of the end plate of the syringe´s

3

plunger part but at the return of the said plunger part springs back while forming a stop for the said end plate. The syringe will then contain correct volume or dose.

Alternatively or in addition the end of the dosage rod may be designed as a driving dog engaging the plunger part´s end plate and then contribute to the displacement of the rod.

In one embodiment the body portion is adapted to permit axial displacement of the bottle relative to the syringe. Then it will be possible to release the bottle from the syringe when the latter is secured on the body portion. Thus, it will be possible to release the bottle from the syringe in order to apply a new bottle without the need of changing the position of the syringe.

The invention shall now be more closely described with reference to the accompanying drawing.

Figure 1 is a vertical section through an embodiment of an instrument according to the invention, the medicament bottle and the syringe being applied in position.

Figure 2 is a plan view of the instrument, however without bottle and syringe.

Figure 3 is a front view of the instrument according to Figure 2.

Figure 4 is a detail view illustrating a modification of the end of the dosage rod coacting with the plunger part of the syringe.

Figure 5, finally, is a detail view illustrating a modification of the end of the instrument which coacts with the bottom of

4

the medicament bottle.

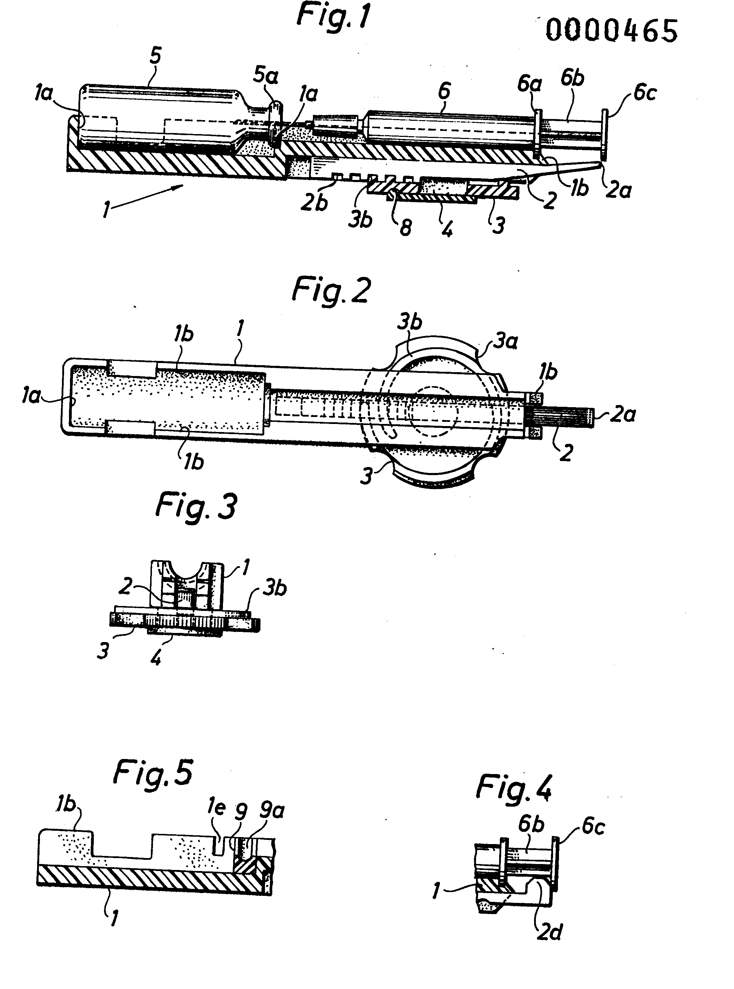

Referring to Figures 1-3 an instrument for filling an injection syringe of non-current or multiple-use type consists of a body portion 1 having at one end a space for positioning a medicament bottle 5 and at the other end a space for positioning an injection syringe 6. To position the medicament bottle the body portion may comprise vertical walls 1a coacting with the bottom and cap, respectively, of the bottle. Alternatively or in addition the bottle may be secured by providing the bottle space of the body portion with resilient side-walls or a separate rear spring (not shown) forcing the bottle towards the wall 1a engaging the cap.

The injection syringe can be secured correspondingly and, further, by hooks or snap members 1b situated at the end of the body portion and engaging a flange 6a on the syringe.

To permit correct setting of the medical dose a dosage rod 2 is displaceably movable in a space at the bottom side of the body portion. In the embodiment shown in Figure 1-3 the end 2a of the dosage rod 2 is laterally resilient and adapted to serve as a stop for an endplate 6c at the syringe's plunger part 6b.

To displace the dosage rod 2 a finger-actuated control knob 3 is journalled at the body portion so that it is rotatable around an axis perpendicular to the axis of the syringe. To improve the gripping-reliability the knob is provided with cuts or indentions 3a. The upper side of the control knob 3 as seen in

5

Figure 1 is provided with a worm 3b coacting with corresponding gears at the bottom side of the dosage rod taking the shape of cuts 2b so that a worm gear is formed. Said worm gear will provide carefully guided displacement of the dosage rod and automatic axial securing thereof in the position set.

In addition the control knob 3 is provided with one or more distinctly noticeable or audible markings per revolution, in the illustrated embodiment accomplished by a ball-latch 8 or the like. A lock plate for the control knob is denoted 4.

The instrument shown in Figures 1-3 shall be used in the following manner:

1. The bottle 5 is placed in its space.

2. Ordinated dose is set by means of the knob 3.

3. The syringe 6 and its needle 6e is brought against the body portion, displaced forwardly and locked by means of the hooks 1b while at the same time the needle 6e has penetrated the membrane cap 5a of the bottle 5.

4. The plunger part 6b is pulled outwardly and the syringe filled.

5. The plunger part is pushed backwards, whereby the end 2a of the dosage rod 2 acts as a stop for the end plate 6c of the plunger part.

In the modified embodiment shown in Figure 4 the end of the dosage rod instead is provided with a driving dog 2d coacting with the end plate 6c. In this embodiment turning of the control knob 3, thus, effects displacement of the plunger

6

part via the worm gear 3b, 2b. Rotation one revolution of the control knob may then accomplish displacement of the dosage rod 2 and, further, the plunger part 6b for instance 4-5 mm. In this embodiment finger-actuation of the plunger part proper will then not take place.

Figure 5 iluustrates and embodiment adapted to permit axial displacement of the bottle 5 so that replacement thereof is permitted while retaining the syringe in its position and without the risk of damage to the needle.

For this purpose the side-walls 1b of the body portion have two or more recesses 1e in which an insert member 9 may be secured in selected position. The member 9 has a slot 9a adapted to coact with a front flange 5a on the bottle 5 (Figure 1). The body portion lacks a rear wall. This arrangement will also permit simple adaption to syringes and bottles of different size and type.

In both embodiments described above at filling the plunger of the syringe may be pulled beyond the actual dosage position and then be pushed back to engage the dosage rod 2. Then it is ascertained that air possibly drawn into the syringe will be removed.

0000465

1

## Claims

1. An instrument for filling an injection syringe of non-recurrent or multiple-use type comprising a body portion (1) having a means (e.g. 1a, 1b) for securing a medicament or drug bottle (6), so that the needle of the syringe precisely hits and penetrates the cap of the bottle and having an axially movable dosage rod (2) which by coaction with the plunger part (6b, 6c) of the syringe (6) permits drawing in a predetermined volume into the syringe, c h a r a c t e r i z e d by a finger-actuated control member (3) which is rotatably journalled at the body portion and via a transmission means (3b, 2b) effects the displacement of the dosage rod (2).

2. An instrument according to claim 1, c h a r a c t e r i z e d in that the control member comprises a knob (3) rotatable around an axis which is perpendicular to the axis of the syringe (6).

3. An instrument according to claim 2, c h a r a c t e r i z e d in that the control knob (3) via a worm gear (3b, 2b) actutates the dosage rod (2).

4. An instrument according to any of claims 1-3, c h a r a c t e r i z e d in that the outer end (2a) of the dosage rod is laterally resilient.

5. An instrument according to any of claims 1-4, c h a r a c t e r i z e d in that the outer end of the dosage rod is formed as a driving dog (2d) for the plunger part (6b, 6c) of the syringe.

2

6.　　　An instrument according to any of claims 1-5, c h a r a c t e r i z e d  in that the body portion (1) is adapted to permit axial displacement of the bottle (5) relative to the syringe (6) when the syringe is secured in the body portion.

Fig.1

0000465

Fig.2

Fig.3

Fig.5

Fig.4

23531

| Category | Citation of document with indication, where appropriate, of relevant passages | Relevant to claim |
|---|---|---|
| A | US − A − 3 844 318 (RAIA) <br> * Figures 1-3; claim 1 * | 1 |
| A | US − A − 3 833 030 (WALDBAUER) <br> * Figures 2-4; claim 1 * | 1 |
| A | US − A − 3 965 945 (ROSS) <br> * Figure 2; claim 1 * | |
| A | US − A − 3 875 979 (HULTS) <br> * Figures 1,2; claim 1 * | 1 |
| A | US − A − 3 840 011 (WRIGHT) <br> * Figures 1-3; claim 1 * | 1 |
| A | FR − A − 1 577 954 (FABRE) <br> * Figure 1; claim * | 1 |
| A | US − A − 2 739 589 (YOCHEM) <br> * Claim 1 * | |

**DOCUMENTS CONSIDERED TO BE RELEVANT**

**CLASSIFICATION OF THE APPLICATION (Int. Cl.²)**

A 61 M 5/00

**TECHNICAL FIELDS SEARCHED (Int.Cl.²)**

A 61 J 1/00
A 61 M 5/00
A 61 M 5/315

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 11-10-1978 | NOESEN |

EPO Form 1503.1 06.78